# EUROPÄISCHE PATENTANMELDUNG

(11) **EP 1 602 384 A1**
(43) Veröffentlichungstag der Anmeldung: **07.12.2005**
(21) Anmeldenummer: 05009266.7
(22) Anmeldetag: 28.04.2005
(51) Int. Cl.: A61M 1/00, A61B 5/20, G01F 19/00

(54) **Anordnung zum Sammeln und Messen von Flüssigkeiten**

(30) Priorität: 02.06.2004 DE 102004027333
(71) Anmelder: PRIMED HALBERSTADT MEDIZINTECHNIK GMBH, 38820 Halberstadt (DE)
(72) Erfinder: Klimenta, Klaus, 38835 Zilly (DE); Träger, Peter, 38820 Halberstadt (DE)
(74) Vertreter: Bock, Gerhard

(57) **Zusammenfassung**

Die Erfindung betrifft eine Anordnung zum Sammeln und Messen von Flüssigkeiten, insbesondere bei der Drainage von Körperflüssigkeiten.

Die Aufgabe der Erfindung, eine einfach konstruierende und leicht zu handhabende Anordnung zum Sammeln und Messen von Flüssigkeiten zu schaffen, die eine Grobmessung bei gleichzeitiger Feinmessung von Flüssigkeitsvolumina ermöglicht, Volumina in der Größenordnung von mindestens 500 ml messgenau erfassen kann und ein Auffangpotential für Flüssigkeiten in der Größenordnung von mindestens 2500 ml gewährleistet, wird dadurch gelöst, dass die Vorrichtung aus einer Grobmesskammer (1) und einer Feinmesskammer (2), die als Teile eines einzigen Bauteils nebeneinanderliegend angeordnet sind, wobei beide Kammern an ihren gleichliegenden durchsichtigen Außenflächen ml-Messskalen (11) und (21) besitzen, besteht, die Feinmesskammer (2) einen Zulauf (9) sowie in ihrer Deckfläche (22) eine Öffnung (23) aufweist, durch die eine hohle, unten offene Stange (24) eines kegelförmigen Kolbens (3), der mit Stange (24) höhenverstellbar ist und mit einer am unteren Ende der Feinmesskammer (2) vorgesehenen konischen Sitzfläche (31) zu Verschlusszwecken zusammenwirkt, die Stange (24) oben durch einen Drehgriff (4) verschlossen ist, während ihr unteres Ende zu einem Auffangbeutel (5) hin offen ist, die Feinmesskammer (2) in ihrem oberen Teil eine Öffnung (6) und die Grobmesskammer (1) eine etwas tiefer liegende Öffnung (7) besitzen, die Öffnung (7) den Überlauf der Feinmesskammer (2) bildet, der in die Grobmesskammer (1) führt, die Öffnung (6) den Überlauf beider Kammer (1) und (2) bildet, der über einen Ablauf (10) in den Auffangbeutel (5) führt.

## Beschreibung

Die Erfindung betrifft eine Anordnung zum Sammeln und Messen von Flüssigkeiten, insbesondere bei der Drainage von Körperflüssigkeiten.

Vorrichtungen und Anordnungen zum Sammeln und / oder Aufbewahren von Körperflüssigkeiten sind schon seit Jahrzehnten auf dem Gebiet der Medizintechnik, bspw. für Anwendungen in der Blutspende oder bei der Drainage im Harnwegsbereich, bekannt, wobei einige dieser Vorrichtungen bzw. Anordnungen ein Messen der Flüssigkeitsvolumina ermöglichen.

Die Schrift DE 38 53 009 T 2 offenbart eine Anordnung zur Sammlung und Messung von Körperflüssigkeiten, aus der ein hohler Ventilkörper bekannt ist, der in eine Aufnahmekammer und eine Überlaufkammer unterteilt ist, wobei die Aufnahmekammer unten eine Auslassöffnung hat, welche in der Schließstellung des Ventilkörpers durch den Ventilsitz verschlossen ist und die an ihrem oberen Teil über mindestens eine Öffnung in der Kammerwand mit dem Messbehälter verbunden ist, wobei die Überlaufkammer an ihrem oberen Ende über eine Öffnung in der Kammerwand mit dem Messgefäß verbunden ist.

Der Erfindung liegt die Aufgabe zugrunde, eine einfach konstruierende und leicht zu handhabende Anordnung zum Sammeln und Messen von Flüssigkeiten zu schaffen, die eine Grobmessung bei gleichzeitiger Feinmessung von Flüssigkeitsvolumina ermöglicht, Volumina in der Größenordnung von mindestens 500 ml messgenau erfassen kann und ein Auffangpotential für Flüssigkeiten in der Größenordnung von mindestens 2500 ml gewährleistet.
Darüber hinaus soll diese Anordnung die Ablesefehler für die Fein- und Grobmessung bei verschiedenen Betrachtungswinkeln des Auges miminieren.

Erfindungsgemäß wird die Aufgabe durch eine Anordnung gemäß Anspruch 1 gelöst. Vorteilhafte Ausgestaltungen sind in den untergeordneten Ansprüchen angegeben.

Die Erfindung wird nachstehend an Hand der schematischen Zeichnungen des Ausführungsbeispiels näher erläutert. Es zeigen:
- Fig. 1:: die schematische Darstellung einer erfindungsgemäßen Anordnung,
- Fig. 2:: die schematische Darstellung eines Detailausschnitts der Anordnung in Fig. 1.

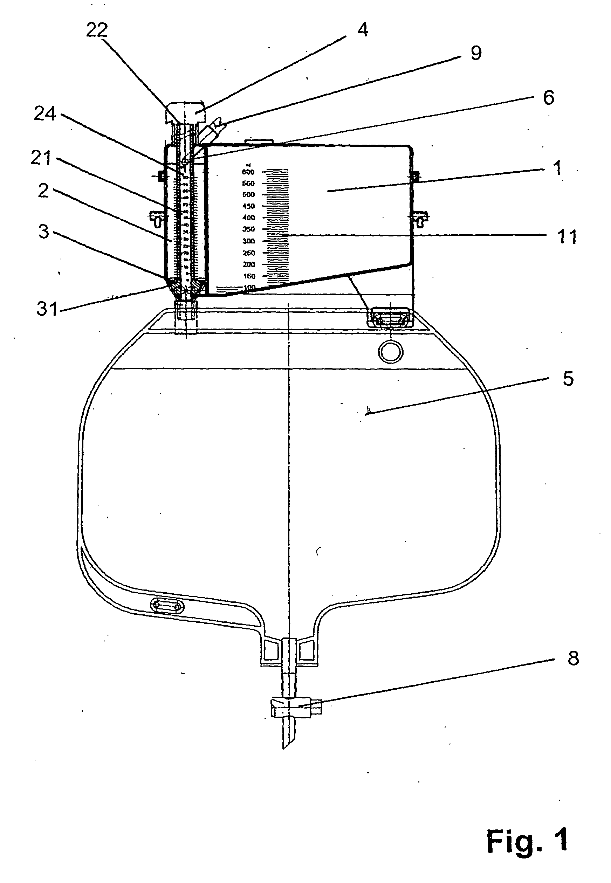

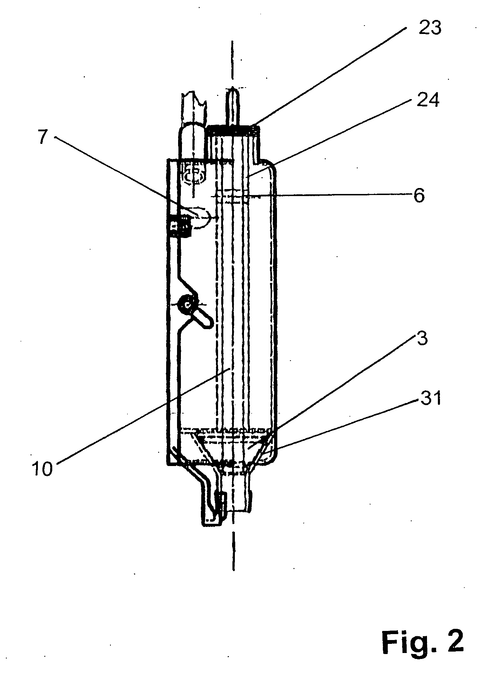

Die Anordnung zum Sammeln und Messen von Flüssigkeiten besteht, wie in den Figuren 1 und 2 beispielhaft dargestellt, aus einer Grobmesskammer (1) und einer Feinmesskammer (2), die als Teile eines einzigen Bauteils nebeneinanderliegend angeordnet sind. Beide Kammern besitzen an ihren gleichliegenden durchsichtigen Außenflächen ml-Messskalen (11) und (21). Die Feinmesskammer (2) besitzt einen Zulauf (9) sowie in ihrer Deckfläche (22) eine Öffnung (23), durch die eine hohle, unten offene Stange (24) eines kegelförmigen Kolbens (3), der mit Stange (24) höhenverstellbar ist und mit einer am unteren Ende der Feinmesskammer (2) vorgesehenen konischen Sitzfläche (31) zu Verschlusszwecken zusammenwirken kann. Die Stange (24) ist oben durch einen Drehgriff (4) verschlossen, während ihr unteres Ende zu einem Auffangbeutel (5), der mit einem Ablasshahn (8) versehen ist, hin offen ist. Diese Feinmesskammer (2) hat in ihrem oberen Teil eine Öffnung (6). Ebenso hat die Grobmesskammer (1) eine etwas tiefer liegende Öffnung (7). Beide Öffnungen (6) und (7) dienen als Überläufe. Die Öffnung (7) bildet den Überlauf der Feinmesskammer (2), der in die Grobmesskammer (1) führt, und die Öffnung (6) bildet den Überlauf beider Kammern (1) und (2), der über einen Ablauf (10) in den Auffangbeutel (5) führt.

Besonders vorteilhaft weißt die Grobmesskammer (1) ein Fassungsvermögen von mindestens 500 ml und die Feinmesskammer (2) ein Fassungsvermögen von mindestens 75 ml auf

Die erfindunggemäße Anordnung besteht besonders vorteilhaft aus Kunststoff oder Plastik, kann jedoch aus Glas o.ä. gefertigt sein.

Bei der Verwendung der erfindungsgemäßen Anordnung gelangt die Körperflüssigkeit über den Zulauf (9) in die Feinmesskammer (2), nach deren vollständiger Befüllung über den Überlauf (7) weiter in die Grobmesskammer (1) und nach deren vollständiger Befüllung über den Überlauf (7) und den Ablauf (10) in den Auffangbeutel (5), der über den Ablasshahn (8) entleert werden kann.
Über den Kolben (3), der mit der Stange (24) höhenverstellbar ist und der in die am unteren Ende der Feinmesskammer (2) vorgesehene konische Sitzfläche (31) greift, ist durch Verdrehen des Drehgriffs (4) ein direktes Entleeren der Grob- und Feinmesskammer (1) und (2) möglich, indem der Kolben (3) von dem Auffangbeutel (5) wegbewegt wird.

Der Vorteil der erfindungsgemäßen Anordnung ist, dass sie eine Grobmessung bei gleichzeitiger Feinmessung von Flüssigkeitsvolumina ermöglich,t ohne dabei die maximal erfassbaren Flüssigkeitsmengen zu gering zu halten.
Gleichzeitig ermöglicht die erfindungsgemäße Anordnung eine Fein- und Grobmessung bei verschiedenen Betrachtungswinkeln des Auges eines Betrachters der Vorrichtung.
Ein Gesamtfassungsvermögen von mindestens 2500 ml liegt im Rahme der Erfindung, wobei die Feinmesskammer 1 ein Volumen von mindestens 75 ml aufweist.

Alle in der Beschreibung, den nachfolgenden Ansprüchen und der Zeichnung dargestellten Merkmale können sowohl einzeln als auch in beliebiger Kombination miteinander erfindungswesentlich sein.

### Bezugszeichen:

- 1: - Grobmesskammer
- 2: - Feinmesskammer
- 3: - Kolben
- 4: - Drehgriff
- 5: - Auffangbeutel
- 6: - Überlauf
- 7: - Überlauf
- 8: - Ablasshahn
- 9: - Zulauf
- 10: - Ablauf
- 11: - ml-Grobmessskala
- 21: - ml-Feinmessskala
- 22: - Deckfläche
- 23: - Öffnung
- 24: - Stange
- 31: - Sitzfläche

## Patentansprüche

1. Anordnung zum Sammeln und Messen von Flüssigkeiten bestehend aus einer Grobmesskammer 1 und einer Feinmesskammer 2, die als Teile eines einzigen Bauteils nebeneinanderliegend angeordnet sind, wobei beide Kammern an ihren gleichliegenden durchsichtigen Außenflächen ml-Messskalen 11 und 21 besitzen,
die Feinmesskammer 2 einen Zulauf 9 sowie in ihrer Deckfläche 22 eine Öffnung 23 aufweist, durch die eine hohle, unten offene Stange 24 eines kegelförmigen Kolbens 3, der mit Stange 24 höhenverstellbar ist und mit einer am unteren Ende der Feinmesskammer 2 vorgesehenen konischen Sitzfläche 31 zu Verschlusszwecken zusammenwirkt,
die Stange 24 oben durch einen Drehgriff 4 verschlossen ist, während ihr unteres Ende zu einem Auffangbeutel 5 hin offen ist,
die Feinmesskammer 2 in ihrem oberen Teil eine Öffnung 6 und die Grobmesskammer 1 eine etwas tiefer liegende Öffnung 7 besitzen,
die Öffnung 7 den Überlauf der Feinmesskammer 2 bildet, der in die Grobmesskammer 1 führt,
die Öffnung 6 den Überlauf beider Kammer 1 und 2 bildet, der über einen Ablauf 10 in den Auffangbeutel 5 führt.

2. Anordnung nach Anspruch 1, **dadurch gekennzeichnet, dass** diese aus Kunststoff und/oder Plastik und/oder Glas besteht.

3. Anordnung nach Anspruch 1, **dadurch gekennzeichnet, dass** die Grobmesskammer 1 ein Fassungsvermögen von mindestens 500 ml besitzt.

4. Anordnung nach Anspruch 1, **dadurch gekennzeichnet, dass** die Feinmesskammer 2 ein Fassungsvermögen von mindestens 75 ml besitzt.

5. Anordnung nach Anspruch 1, **dadurch gekennzeichnet, dass** der Auffangbeutel 5 mit einem Ablasshahn 8 versehen ist.
